(19) Europäisches Patentamt
European Patent Office
Office européen des brevets

(11) Veröffentlichungsnummer: **0 254 220 B1**

(12) # EUROPÄISCHE PATENTSCHRIFT

(45) Veröffentlichungstag der Patentschrift: **12.09.90**

(51) Int. Cl.[5]: **C07C 22/02**, C07C 17/33, C07C 63/72

(21) Anmeldenummer: **87110350.3**

(22) Anmeldetag: **17.07.87**

(54) **Verfahren zur Herstellung von 2.2-Bisphenylhexafluorpropan.**

(30) Priorität: **23.07.86 DE 3624814**

(43) Veröffentlichungstag der Anmeldung: **27.01.88 Patentblatt 88/4**

(45) Bekanntmachung des Hinweises auf die Patenterteilung: **12.09.90 Patentblatt 90/37**

(84) Benannte Vertragsstaaten: **BE CH DE FR GB IT LI NL**

(56) Entgegenhaltungen: **US-A- 3 310 573**

(73) Patentinhaber: **HOECHST AKTIENGESELLSCHAFT, Postfach 80 03 20, D-6230 Frankfurt am Main 80(DE)**

(72) Erfinder: **Schneider, Klaus-Albert, Dr., Schillerring 30, D-6234 Hattersheim am Main(DE)**
Erfinder: **Siegemund, Günter, Dr., Frankfurter Strasse 21, D-6238 Hofheim am Taunus(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

2.2-Bisphenylhexafluorpropan ist die Verbindung der Formel

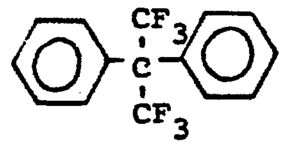

Sie ist hauptsächlich ein wertvolles Zwischenprodukt auf dem Polymerengebiet. Durch Funktionalisierung (Nitrierung und Reduktion der Nitro- zu Aminogruppen) und Kondensation z.B. mit aromatischen Tetracarbonsäuren oder deren Anhydriden gelangt man zu Polyamiden mit hoher chemischer und thermischer Beständigkeit. Ein beispielhaftes Formelschema für eine derartige Kondensation ist:

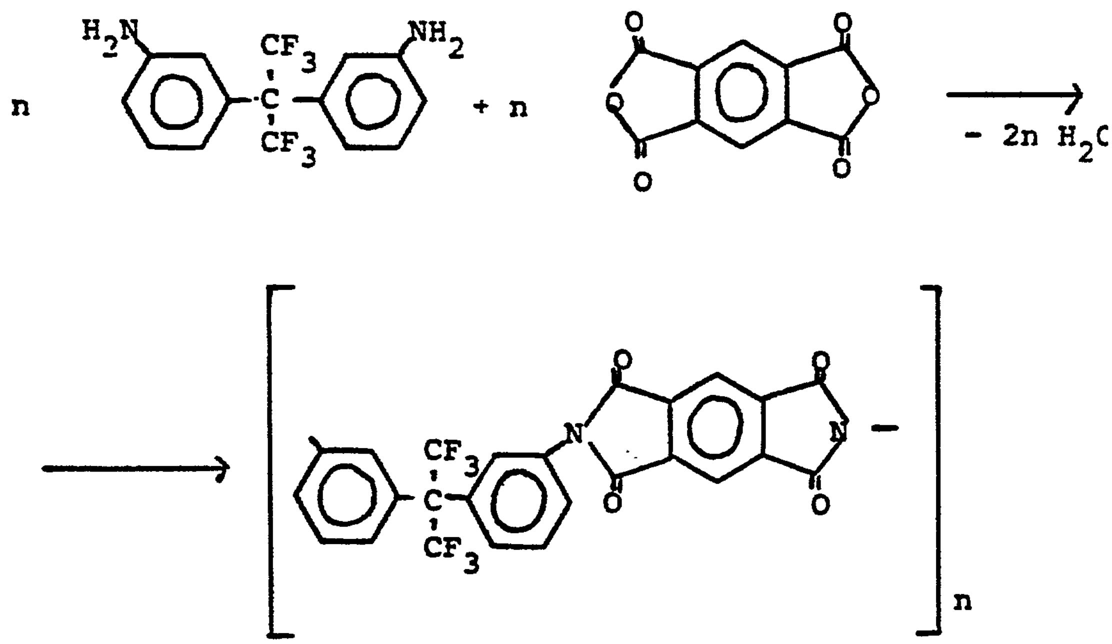

Es ist bekannt, 2.2-Bisphenylhexafluorpropan in einem 2-Stufenprozeß, ausgehend von 2.2-Bis-(4-hydroxyphenyl)-hexafluorpropan, herzustellen (K.S.Y. Lau et al., Journal of Polymer Science, Polymer Chemistry Edition, Vol. 20, S. 2381-2393 (1982)). Bei diesem Verfahren wird 2.2-Bis-(4-hydroxyphenyl)-hexafluorpropan zuerst mit Trifluormethansulfonsäureanhydrid zu 2.2-Bis-(4-triflatophenyl)-hexafluorpropan umgesetzt, welches dann in der 2. Verfahrensstufe katalytisch hydriert wird. Formelmäßig läßt sich das Verfahren wie folgt wiedergeben:

1. Stufe:

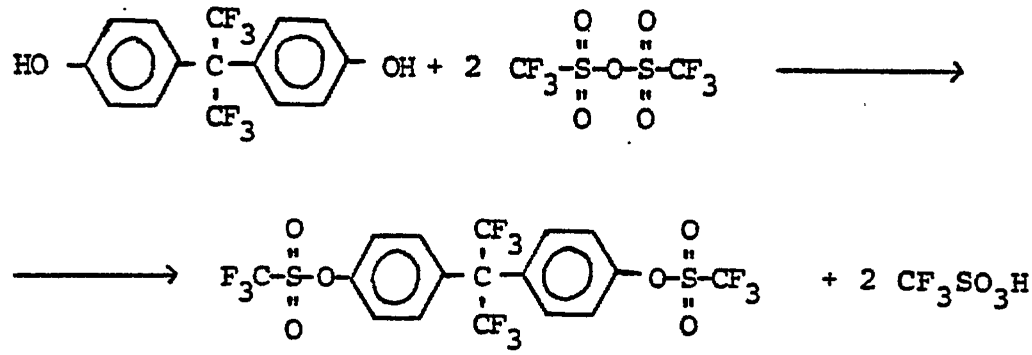

2. Stufe:

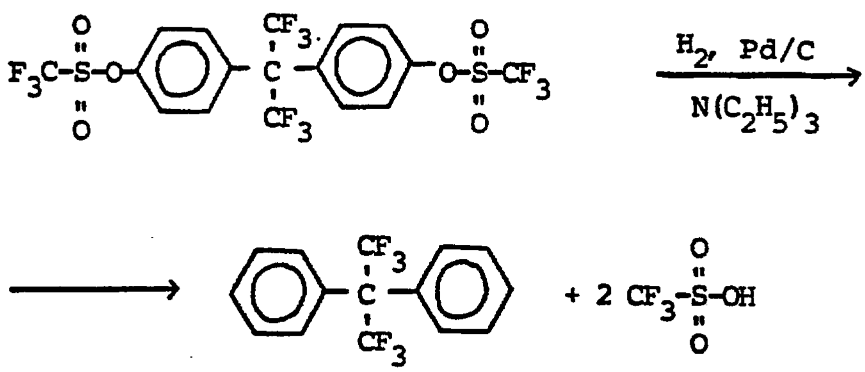


Nach den Ausführungsbeispielen in der genannten Literaturstelle beträgt die Ausbeute in der 1. Stufe 96,5 % und in der 2. Stufe 87,6 % d.Th.

Die Ausgangsverbindung für das Verfahren - 2.2-Bis-(4-hydroxyphenyl)-hexafluorpropan - kann z.B. durch Umsetzung von Hexafluoraceton mit Phenol in flüssigem Fluorwasserstoff erhalten werden (EP-B-0054227).

Das von K.S.Y. Lau et al. (a.a.O.) beschriebene Verfahren liefert zwar hohe Ausbeuten, ist aber insbesondere wegen der Notwendigkeit des Einsatzes des teuren und nur unter erheblichen Sicherheitsvorkehrungen handhabbaren Trifluormethansulfonsäureanhydrids für die technische Durchführung nur wenig geeignet.

In dem Bestreben, ein verbessertes und auch in technischem Maßstab einfach durchführbares Verfahren zur Herstellung von 2.2-Bisphenylhexafluorpropan bereitzustellen, wurde nun gefunden, daß dieses Ziel dadurch erreicht wird, daß man von 2.2-Bis-(4-methylphenyl)-hexafluorpropan ausgeht, diese Verbindung zur entsprechenden Dicarbonsäure oxidiert und die Dicarbonsäure decarboxyliert.

Erfindungsgegenstand ist daher ein Verfahren zur Herstellung von 2.2-Bisphenylhexafluorpropan, das dadurch gekennzeichnet ist, daß man

a) 2.2-Bis-(4-methylphenyl)-hexafluoropropan zu 2.2-Bis-(4-carboxyphenyl)-hexafluorpropan oxidiert und

b) das so erhaltene 2.2-Bis-(4-carboxyphenyl)-hexafluorpropan zu 2.2-Diphenylhexafluorpropan decarboxyliert.

Das Verfahren stellt sich formelmäßig wie folgt dar:

a)

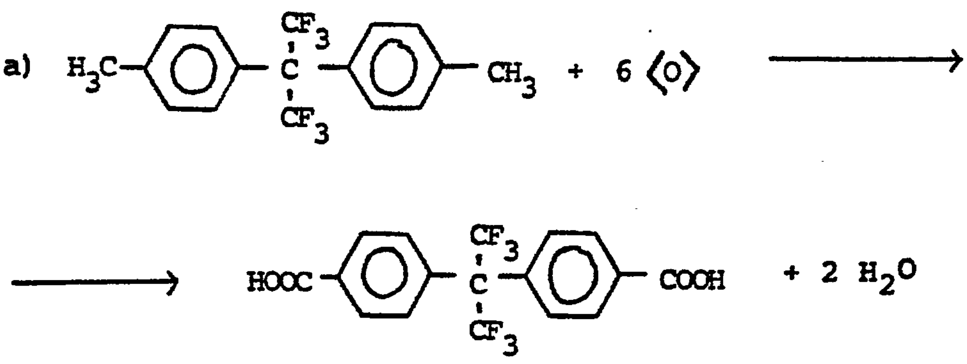

b)

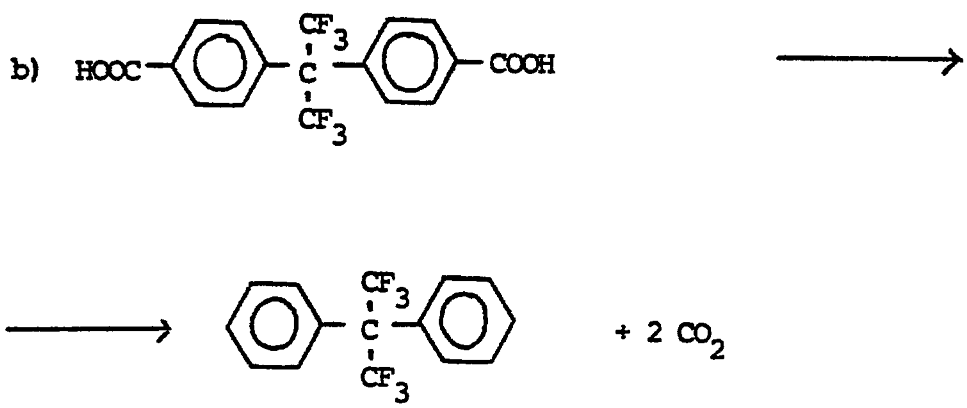

Stufe a) ist zwar aus der Literatur bekannt; vgl. z.BL. Livšic et al., Ž. vsev. chim. Obšc. 11 (1966) Nr. 4, S. 469-470 (Oxydation von 2.2-Bis(4-methylphenyl)hexafluorpropan zu 2.2-Bis(4-carboxyphenyl)hexafluorpropan mit 20%iger Salpetersäure - Ausbeute 82 %), sowie US-A-3 310 573 (Oxydation mit $CrO_3$).

Die Decarboxylierung von 2.2-Bis(4-carboxyphenyl)hexafluorpropan-Stufe b) ist noch nicht beschrieben worden; es ist jedoch bekannt, aromatische Carbonsäuren z.B. in Gegenwart eines Kupferkatalysators in Chinolin zu decarboxylieren - vgl. Fieser & Fieser, Lehrbuch der organischen Chemie, 4. Aufl. (1960), S. 649, Absatz 2.

Die Kombination der beiden Verfahrensstufen a) und b) war jedoch nicht bekannt und ist auch in keiner Weise etwa na heliegend, weil man aufgrund der - immerhin ziemlich neuen, aus dem Jahr 1982 stammenden - Arbeit von K.S.Y. Lau et al. (a.a.O.) annehmen mußte, daß man 2.2-Bisphenylhexafluorpropan nicht auf einfache Weise (wie Oxidation und Decarboxylierung), sondern nur - unter Einsatz teurer und gefährlicher Chemikalien wie Trifluormethansulfonsäureanhydrid - über eine ziemlich komplizierte Zwischenverbindung (2.2-Bis-(4-triflatophenyl)hexafluor-propan!) herstellen kann.

Die Ausbeuten nach dem erfindungsgemäßen Verfahren liegen in der Größenordnung derjenigen des von K.S.Y. Lau et al. beschriebenen Verfahrens. Wegen der Einfachheit der Verfahrensstufen, bei denen keine teuren und gefährlichen Reagentien eingesetzt werden müssen, stellt das - auch in technischem Maßstab leicht durchführbare - Verfahren einen erheblichen Fortschritt gegenüber dem bekannte Verfahren dar.

Die Ausgangsverbindung für das erfindungsgemäße Verfahren - 2.2-Bis-(4-methylphenyl)hexafluorpropan - ist z.B. erhältlich durch Umsetzung von Hexafluoraceton mit Toluol in flüssigem Fluorwasserstoff (B.L. Livsic et al., a.a.O.).

Die Verbindung wird gemäß Verfahrensstufe a) zu der entsprechenden Dicarbonsäure - 2.2-Bis(4-carboxyphenyl)hexafluorpropan - oxidiert wie dies von der Herstellung aromatischer Carbonsäuren durch Oxidation von Methylaromaten her bekannt ist. Die Oxidation kann z.B. mit Salpetersäure oder mit $CrO_3$ entsprechend den beiden vorerwähnten Literaturstellen B.L. Livsic et al. (a.a.O.) und US-A-3 310 573 geschehen. Bevorzugt ist im vorliegenden Fall die Oxidation mit $CrO_3$.

Die Decarboxylierung gemäß Verfahrensstufe b) kann im Prinzip ebenfalls auf bekannte Weise erfolgen; bevorzugt ist hier die Decarboxylierung in Gegenwart einer schwer flüchtigen organischen Stickstoffbase sowie gegebenenfalls zusätzlich noch eines Kupferkatalysators. Als Kupferkatalysator wird bevorzugt Kupferpulver oder ein Gemisch von Kupferpulver und Kupferchromit mit einem geringen Mangangehalt ($CuCr_2O_4$ + ca. 2 % Mn) verwendet. Die Katalysatormenge ist im Prinzip nicht kritisch; vorteilhaft liegt sie jedoch zwischen etwa 0,5 und 5 Gew.-%, bezogen auf die Ausgangs-Dicarbonsäure.

Die Decarboxylierung wird vorzugsweise bei Temperaturen über etwa 200°C, insbesondere bei etwa 200 bis 250°C, durchgeführt. Wegen dieser relativ hohen Temperaturen können als organische Stickstoffbasen natürlich nur solche Stickstoffbasen eingesetzt werden, die bei den Decarboxylierungstemperaturen nicht oder nur wenig flüchtig sind. Als solche organischen Stickstoffbasen kommen z.B. Chinolin, Isochinolin, Chinazolin oder Chinoxalin sowie deren Methyl- oder Chlorderivate in Frage; bevorzugt ist jedoch Chinolin. Die Stickstoffbasen dienen bevorzugt auch als Lösungsmittel.

Nach beendeter Reaktion wird der Ansatz auf an sich bekannte Weise aufgearbeitet.

Das folgende Beispiel soll der näheren Erläuterung der Erfindung dienen.

Beispiel:

a) Oxidation von 2.2-Bis(4-methylphenyl)hexafluorpropan:

664 g (2 mol) 2.2-Bis-(4-methylphenyl)-hexafluorpropan wurden in 6000 ml Eisessig bei 80°C portionsweise mit 1500 g (15 mol) Chromtrioxid versetzt. Über Nacht wurde bei 80 - 90°C nachgerührt, dann noch 2 Stunden unter Rückfluß erhitzt. Anschließend wurde der Eisessig im Vakuum weitgehend abgezogen. Dann wurden ca. 3 l Wasser zugesetzt und die Lösung einer Wasserdampfdestillation un terworfen, um so die gesamte Essigsäure zu entfernen.

Nach dem Abkühlen wurde die Reaktionsmischung filtriert und der Filterkuchen mit Wasser gewaschen. Anschließend wurde der Filterkuchen in heißer Soda-Lösung gelöst und von unlöslichen Resten durch eine Filtration befreit. Durch Ansäuern des Filtrats mit Schwefelsäure wurde die Dicarbonsäure in Freiheit gesetzt. Nach dem Waschen mit wenig Wasser wurden so 621 g 2.2-Bis-(4-carboxyphenyl)-hexafluorpropan (79 % Ausbeute) erhalten.

b) Decarboylierung von 2.2-Bis(4-carboxyphenyl)hexafluorpropan:

Eine Suspension von 417 g (1.06 Mol) 2.2-Bis(4-carboxyphenyl)hexafluorpropan, 1200 ml Chinolin und 10 g Cu-Bronze sowie 5 g $CuCr_2O_4$ + 2 % Mn wurden so lange unter Rückfluß erhitzt, bis kein Kohlendioxid mehr in der Abluft nachweisbar war. Nach dem Abtrennen der festen Bestandteile durch Filtration wurde die Reaktionsmischung mit halbkonzentrierter Salpetersäure angesäuert und anschließend 5 × mit Ether extrahiert. Die vereinten organischen Phasen wurden neutral gewaschen und über $MgSO_4$ getrocknet. Das nach dem Abziehen des Lösungsmittels erhaltene Rohprodukt wurde mittels einer Feststoff-Destillation gereinigt (Sdp. 75°C/0.4 T; Schmp. 31 - 33°C). Die 275 g isolierte Substanz entsprechen einer Ausbeute von 85 % d.Th.

IR (KBr): $\gamma$ = 1300 - 1140 $cm^{-1}$ ($CF_3$).

$^1H$ ($CDCl_3$): $\delta$ = 7.4 (s, breit, 10 H, aromat.).

$^{13}C$ ($CDCl_3$): $\delta$ = 133.8, 130.3, 129.0, 128.3 (aromat.), 124.5 (q, $^1J_{C,F}$ = 286 Hz, $CF_3$, 64.9 (mc, C ($CF_3)_2$).

$^{19}F$ ($CDCl_3$): $\delta$ = -64.1 ($CF_3$).

**Patentansprüche**

1. Verfahren zur Herstellung von 2.2-Diphenylhexafluorpropan,
dadurch gekennzeichnet, daß man
a) 2.2-Bis-(4-methylphenyl)hexafluorporpan zu 2.2-Bis-(4-carboxyphenyl)-hexafluorpropan oxidiert und
b) das so erhaltene 2.2-Bis-(4-carboxyphenyl)hexafluorpropan zu 2.2-Bisphenylhexafluorpropan decarboxyliert.

2. Verfahren nach Anspruch 1, dadurch gekennzeichnet, daß man die Oxidation gemäß Stufe a) mit Chromtrioxid durchführt.

3. Verfahren nach den Ansprüchen 1 - 2, dadurch gekennzeichnet, daß man die Decarboxylierung gemäß Stufe b) in Gegenwart einer schwer flüchtigen organischen Stickstoffbase sowie gegebenenfalls zusätzlich eines Kupferkatalysators durchführt.

4. Verfahren nach den Ansprüchen 1 - 3, dadurch gekennzeichnet, daß man die Decarboxylierung bei Temperaturen über etwa 200°C, vorzugsweise zwischen etwa 200 und 250°C, durchführt.

## Claims

1. A process for preparing 2,2-diphenylhexafluoropropane, which comprises
a) oxidizing 2,2-bis-(4-methylphenyl)-hexafluoropropane to 2,2-bis-(4-carboxyphenyl)-hexafluoropropane and
b) decarboxylating the resulting 2,2-bis-(4-carboxy-phenyl)-hexafluoropropane to 2,2-bisphenyl-hexafluoropropane.

2. The process as claimed in claim 1, wherein the oxidation according to stage a) is carried out with chromium trioxide.

3. The process as claimed in claim 1 or 2, wherein the decarboxylation according to stage b) is carried out in the presence of an organic nitrogen base of low volatility and, optionally in addition of a copper catalyst.

4. The process as claimed in any of claims 1–3, wherein the decarboxylation is carried out at temperatures above about 20°C, preferably between about 200 and 250°C.

## Revendications

1. Procédé de préparation du 2,2-diphénylhexafluoropropane, procédé caractérisé en ce que:
a) on oxyde le 2,2-bis-(4-méthylphényl)hexafluoropropane en 2,2-bis (4-carboxyphényl)-hexafluoropropane, et
b) on décarboxyle celui-ci pour obtenir le 2,2-bisphényl-hexafluoropropane.

2. Procédé selon la revendication 1, caractérisé en ce que l'on effectue l'oxydation de l'étape a) avec du trioxyde de chrome.

3. Procédé selon la revendications 1 ou 2, caractérisé en ce que l'on effectue la décarboxylation de l'étape b) en présence d'une base organique azotée peu volatile ainsi qu'éventuellement un catalyseur au cuivre.

4. Procédé selon les revendications à 1–3, caractérisé en ce que l'on effectue la décarboxylation à des températures supérieures à 200°C, de préférence entre 200 et 250°C.